# EUROPEAN PATENT APPLICATION

(11) **EP 1 418 231 A1**
(43) Date of publication of application: **12.05.2004**
(21) Application number: 02079675.1
(22) Date of filing: 08.11.2002
(51) Int. Cl.: C12N 9/96, C12N 11/00, C12N 9/04, C12N 9/02

(54) **Stabilised crosslinked enzyme aggregates**

(71) Applicant: Avantium International B.V., 1014 BV Amsterdam (NL)
(72) Inventor: Smith, Alan Arthur, 2011 ZL Haarlem (NL); Wegman, Margaretha Anna, 2622 JN Delft (NL)
(74) Representative: Wittop Koning, Tom Hugo

(57) **Abstract**

Described is a method for the preparation of crosslinked enzyme aggregates, comprising the steps of:
A - providing a plurality of enzyme molecules,
B - aggregating the enzymes molecules in a liquid medium, by means of addition of a precipitating agent,
C - crosslinking the aggregated enzymes to one another by providing a crosslinking agent in the liquid medium, the method comprising, during or between steps A, B or C, the step of adding an enzyme activity enhancing agent, interacting with the said enzyme molecules.

The resulting CLEA are advantageous due to increase in activity, their suitability for recycle, enhanced stability and low requirements for additional enzyme activity enhancing agent. Further, crosslinked enzyme aggregates are described, comprising an enzyme activity enhancing agent associated therewith.

## Description

The present invention relates to crosslinked enzyme aggregates of cofactor dependent enzymes, comprising the steps of:
A) providing a plurality of cofactor dependent enzyme molecules,
B) aggregating the cofactor dependent enzymes in a liquid medium, by means of addition of a precipitating agent, and
C) crosslinking the aggregated cofactor dependent enzymes to one another by providing a crosslinking agent in the liquid medium.

Methods for the preparation of crosslinked enzyme aggregates (further also abbreviated as CLEAs) are known in the art and comprise e.g. steps of aggregating the enzymes in a liquid medium, comprising a precipitating agent and crosslinking the aggregated enzymes to one another by providing a crosslinking agent in the liquid medium.

A method for the preparation of CLEAs of the above-mentioned type is known from e.g. EP 1 088 887, herein incorporated by reference.

Crosslinked enzyme aggregates can be regarded as self-supported immobilised enzymes, resulting in a final preparation with a high concentration of enzyme per unit volume and having potentially broad applicability (L. Cao et al., Org. Lett., 2000, 2, 1301-1304; L. Cao et al., J. Mol. Catal. B: Enzymatic, 2001, 11, 665-670, P. L6pez-Serrano et al., Biotechnol. Lett., 2002, 24, 1379-1383). Crosslinked enzyme crystals (CLECS) can also be regarded as self-supported immobilised enzymes. However, to immobilise enzyme crystals via crosslinking, an adequate crystallisation protocol needs to be developed by a skilled person in the art, including enzyme purification and crystallisation itself, wherein especially the crystallisation is time-consuming, laborious and not straightforward.

Crosslinked enzyme aggregates can advantageously be used as industrial biocatalysts, for analytical and other research purposes. In the said fields, enzymes, or biocatalysts, are often immobilised on a solid carrier, leading to a so called "dilution of activity", because in general, the enzyme only constitutes 5% of the weight of the immobilised mass; 95% or more thereof is constituted by the non-catalytic material.

In the art, CLEAs are prepared by precipitating the enzymes of interest by a precipitating agent, such as inorganic salts, e.g. ammonium sulphate, organic polymers, such as poly(ethylene glycol) or organic solvents, such as tert-butyl alcohol. However, the skilled person is aware of additional suitable precipitating agents, useable for aggregating the enzymes. Also, CLEAs are prepared wherein precipitation and/or crosslinking is performed in the presence of a compound chosen from the group of a) a crown ether and b) a surfactant. The conditions for aggregating and crosslinking the enzymes are e.g. described in the art (Cao et al., P. López-Serrano et al., Supra) and are common knowledge in the art. There are no restrictions for enzymes to be used for immobilisation in the form of CLEAs, as long as the enzymatic activity is not hampered significantly by the aggregation and crosslinking process. For a skilled person, straightforward experiments will give an indication about the suitability of the enzyme of interest to be immobilised by the CLEA technique. One of the advantages of CLEAs is the fact that they can be prepared in the absence of solid carrier, although a carrier can be used if desired.

However, the activity of many enzymes can be enhanced or facilitated by the presence of additional molecules, interacting with the enzyme. Herein, the term "enzyme activity enhancing agent" is defined as such a factor. The term "interacting with the enzyme" is defined as the factor being associated with the enzyme, such as bound to the enzyme, or captured in a molecular cavity or pocket of the enzyme molecule, wherein this association leads to enhanced activity of the enzyme. The term "enhanced activity" is meant to encompass also the improvement of enzyme stability, as an enzyme, stabilised this way, will have a prolonged activity. Examples of such enzyme activity enhancing agents are e.g. cofactors, such as NADH, or molecules, captured in the pockets of the enzymes, leading to improved stability, such as dimethylsulfoxide (DMSO). With the above definitions, the skilled person will be able to identify additional enzyme activity enhancing agents.

In the art, it has however been difficult to immobilise enzymes with their corresponding enzyme activity enhancing agents. For example, several attempts have been made to prepare CLEAs of cofactor dependent enzymes (Cao et al., P. López-Serrano et al., Supra), however resulting in CLEAs without or having low activity. Immobilisation of cofactor dependent enzymes to a solid carrier has not led to a solution since besides dilution of activity a significant loss of activity is observed. (Keinan et al., Ann. N.Y. Acad. Sci., 1987, 501, 130-149; Gorrebeeck et al., Rec. Trav. Chim. Pays-Bas, 1991, 110, 231-235; Tarhan et al., Proc. Biochem. 1995, 30, 49-55).

CLEAs of enzymes, associated with enzyme activity enhancing agents, such as cofactors, can be used in a plurality of biochemical processes on industrial or laboratory scale, for the preparation of compounds through a redox enzymatic process. Non-limiting examples thereof are the preparation of alcohols, ketones, aldehydes, amino acids, hydroxy carboxylic acids.

The present invention now provides a method for the preparation of CLEAs of enzymes associated with enzyme activity enhancing agent, which are highly active, wherein the method comprises, during or between steps A, B or C, the step of adding an enzyme activity enhancing agent, interacting with the said enzyme molecules.

Thus, the enzyme activity enhancing agent is contacted with the enzyme and enabled to associate therewith before crosslinking of the enzyme takes place. This way, the enzyme activity enhancing agent is "captured" by the enzyme at the proper interaction site, resulting in an enzyme of enhanced activity, and the enzymes are crosslinked to one another having the enzyme activity enhancing agent associated therewith. According to the method of the invention, CLEAs are prepared, wherein the enzyme activity enhancing agent is stably incorporated by association with the enzyme molecules. It has also been found that the enzyme activity enhancing agent is stably present in the CLEAs such, that also during incubation of the CLEAs in liquid media, the enzyme activity enhancing agent does not leak out of the CLEAs, or at least significantly less as compared with corresponding CLEAs prepared according to the art. Without being bound to any explanation, it is believed that when CLEAs are prepared without the enzyme activity enhancing agent according to the invention, a conformational change of the enzyme molecules may take place during the crosslinking step, resulting in an inactive conformation, being unable to associate with the enzyme activity enhancing agent.

The enzyme activity enhancing agent may physically or covalently be bound to the enzyme. In case of physical binding of the enzyme activity enhancing agent to the enzyme activity enhancing agent enzyme, the enzyme activity enhancing agent may be added as a solid, dissolved in an aqueous medium, suspended or dissolved in an organic solvent, wherein the addition may be at once or slowly.

The enzyme activity enhancing agent is preferably added before step C), as during step C), crosslinking may interfere with the association of the enzyme activity enhancing agent with the enzyme molecules, resulting in less incorporation of enzyme activity enhancing agent in the CLEAs, and thus to less active CLEAs. The enzyme activity enhancing agent can be combined with the enzyme molecules in step A), or added before or together with the precipitating agent, or thereafter. The skilled person will for each envisaged CLEA to be prepared, be able to easily determine the optimal moment for addition of the enzyme activity enhancing agent. It is found that in the presence of a stabiliser as enzyme activity enhancing agent, the conformation of the enzyme is stabilised and that the activity of the enzyme is significantly improved compared to corresponding CLEAs, prepared without the enzyme activity enhancing agent.

Preferably, the enzyme molecules comprise cofactor dependent enzymes; according to the invention, CLEAs can be prepared wherein such enzymes are associated with their corresponding cofactors.

Cofactor dependent enzymes are preferably chosen from a group of oxidoreductases, which comprise all cofactor dependent enzymes of the first main division of the Enzyme Commission of International Union of Biochemistry and Molecular Biology (abbreviated as EC). The EC classification system has been described by Dixon, Webb, Thorine and Tipton ("Enzymes", Chapter 5, pages 207-230, Academic Press, 1979). Oxidoreductases all are classified as EC 1.x.y.z that mediate the transfer of electrons, H atoms, or hydride atoms, wherein x relates to the subclasses of enzymes specified by the first digit, which pertains to a group of twenty subclasses of functional groups of the substrates and/or products for the enzyme, y relates to a series of sub-subclasses and z specifies a serial number for the enzyme, which is often related to the particular identity of the natural substrate of the enzyme. The cofactor dependent enzymes are preferably chosen from the group of alcohol dehydrogenases (EC 1.1.y.z), formate dehydrogenase (1.2.1.z), L-alanine dehydrogenase (EC 1.4.1.1), L-leucine dehydrogenase (1.4.1.9) as is used by Degussa (Germany) for the production of amino acids (A. Liese, K. Seelbach, C. Wandrey, Industrial Biotransformations, Chapter 5, pages 125- 128, Wiley-VCH, 2000), the alcohol dehydrogenases preferably derived from *Lactobacillus kefir,* horse liver, *Lactobacillus brevis, Candida boidinii, Candida parapsilosis, Rhodococcus erythropolis,* yeast, *Thermoanaerobium brockii, Neurospora crassa, Acinetobacter calcoaceticus, Zygosaccharomyces rouxii, Candida sorbophila,* the formate dehydrogenases preferably derived from *Candida boidinii, Pseudomonas* spp., yeast, the L-alanine dehydrogenase preferably from *Bacillus* cereus, the L-leucine dehydrogenase derived from *Bacillus cereus, Bacillus sphaericus.*

Regarding the above, the enzyme activity enhancing agent preferably comprises a cofactor. As outlined above, however, the method according to the invention can also be used to prepare CLEAs, comprising enzymes, associated with stabilising agents, such as DMSO, e.g. known for the stabilising effect on alcohol dehydrogenases, such as horse liver alcohol dehydrogenase.

The enzyme activity enhancing agent is preferably chosen from the group of cofactors, such as Flavin adenine dinucleotide (FAD), acetyl coenzyme A, Adenosine 5'-monophosphate (AMP), Adenosine 5'-diphosphate (ADP), Adenosine 5'-triphosphate (ATP), S-(5'-Adenosyl)-L-methionine (SAM), HS-coenzyme A, or more preferably of nicotinamide cofactors, such as β-Nicotinamide adenine dinucleotide (NAD⁺, NADH), β-Nicotinamide adenine dinucleotide phosphate (NADP⁺, NADPH), or a mixture thereof. Alternatively, a nicotinamide cofactor analogue could be chosen, including but not limited to the examples of NADH being an analogue of NADPH and NAD⁺ covalently attached to watersoluble polymers.

All different combinations of enzymes and enzyme activity enhancing agents are possible, preferably the combination of a cofactor dependent enzyme and a enzyme activity enhancing agent enzyme activity enhancing agent, wherein the enzyme activity enhancing agent is preferably a cofactor required for the enzyme activity, more preferably combinations of horse liver alcohol dehydrogenase and NADH, *Lactobacillus kefir* alcohol dehydrogenase and NADPH, however this list is not extensive. Those who are skilled in the art will easily be able to provide and identify suitable combinations of enzymes and enzyme activity enhancing agents.

The crosslinking agent may be any suitable crosslinking agent, known in the art, such as glutaraldehyde. Glutaraldehyde is known to be a very potent crosslinking agent for peptides and proteins. In a preferred embodiment, the crosslinking agent being prepared by combining a first and a second compound each having at least two reactive groups, the reactive groups of the first compound being primary amino groups, the reactive groups of the second compound being aldehydes groups, e.g. as described in PCT/NL02/00176 (Cao et al.), herein incorporated by reference.

The present invention is not limited to CLEAs of single enzymes, but also to so-called multi-CLEAs, preferably of cofactor dependent enzymes. Thereto, preferably at least two different enzymes, preferably cofactor dependent enzymes are provided in step A) and at least one, preferably more than one, enzyme activity enhancing agents are added during or between steps A), B) or C). This way, multiple enzyme CLEAs (multi-CLEAs) can be prepared.

A not limiting example of a combination of multi-CLEAs of cofactor dependent enzymes is formate dehydrogenase/alcohol dehydrogenase or alcohol dehydrogenase/hydrogenase (EC 1.12). However, the skilled person is aware of additional suitable combinations of enzymes/cofactor dependent enzymes to prepare multi-CLEAs, e.g. for conversion of a main substrate. The ratios of the enzymes in the multi-CLEA systems may be optimized for a particular application, as will be appreciated in the art.

Preferably, steps B) and C) are performed simultaneously; it has been found that CLEAs having high activity can be produced by this simplified process. In this case, the enzyme activity enhancing agent is preferably added before the combined steps B) and C), although it can also be added simultaneously to these steps. CLEAs are preferably used as biocatalyst in biological or biochemical reactions. As these reactions often involve redox reactions, wherein the enzyme is associated with a cofactor, CLEAs, in particular multi-CLEAs, prepared according to the present invention can advantageously be used in such reactions.

When a cofactor dependent enzyme is used, the oxidation state of the cofactor usually changes during a redox reaction. It is therefore desired to regenerate the cofactor since it is expensive when it is used in stoichiometric amounts. It has been found that CLEAs, prepared according to the invention, comprise these cofactors stably incorporated and are not easily washed out. Therefore, the CLEAs according to the invention can be recycled, without significant loss of any cofactor, present in the said CLEAs. Such recycling can e.g. be performed by performing a second redox reaction to allow the cofactors in the CLEAs to regenarate into their original state. Accordingly, the method according to the invention preferably further comprises the steps of:
D) performing an enzyme catalysed reaction in a liquid medium in the presence of crosslinked enzyme aggregates, obtained in step C), and
E) recycling the crosslinked enzyme aggregates of step D).

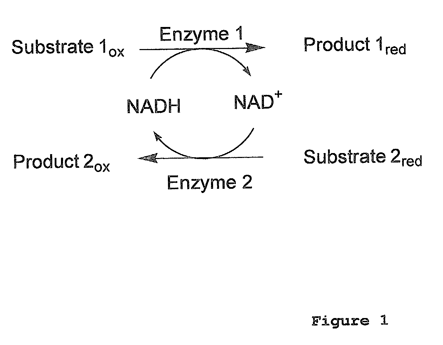

In a very attractive embodiment however, cofactor regeneration can take place via a coupled-enzyme process. Thereto, a multi-CLEA as described above is used, wherein at least one cofactor is present that is active in the oxidised state for a first enzyme, present in the CLEA, and active in the reduced state for a second enzyme, present in the same CLEA. This way, the cofactor is recycled for the first enzyme by a second redox reaction, catalysed by the second enzyme, so that the cofactor can re-enter the reaction cycle (Figure 1). This reaction cycle shows a coupled-enzyme process, in which two parallel redox reactions (conversion of the main substrate and cofactor recycling) are catalysed by two different cofactor dependent enzymes as is known in the art (Seelbach, K., Riebel, B., Hummel, W., Kula, M.-R., Tishkov, V. I., Egorov, A. M., Wandrey, C. and Kragl, U. (1996) A novel, efficient regeneration method of NADH using a new formate dehydrogenase, Tetrahedron Lett. 37 (9) 1377-1380).

According to this embodiment, steps D) and E) are combined and in step D), at least two enzyme catalysed reactions are performed, the crosslinked enzyme aggregates comprising at least two cofactor dependent enzymes, the first enzyme being active in catalysing the first reaction when associated with the cofactor in oxidised state, the second enzyme being active in catalysing the second reaction when associated with the said cofactor in reduced state.

The invention further relates to crosslinked enzyme aggregates, obtainable with the method according to the present invention, and to crosslinked enzyme aggregates comprising an enzyme activity enhancing agent associated therewith.

With such CLEAs, enhanced thermal stabilization of the enzyme system can be achieved so that the enzyme may be used at higher temperatures for longer periods than the native enzyme. Further, the enzyme may give enhanced chemical stabilization of the enzyme system such that the enzyme may be used in non-physiological environments , such as in organic solvent, for longer periods than the native enzyme, or at high or low pH, for longer periods than the native enzyme. The CLEAs having enhanced stability can also be used to allow reduction of the biocatalytic mass necessary for a given transformation.

The CLEAs according to the invention are, as indicated above, recyclable and can be reused without the need for additional exogenous cofactor in biotransformation processes.

The CLEAs according to the invention may also have improved enzyme activity and/or selectivity over the native preparation, i.e. CLEAs, prepared without enzyme activity enhancing agent.

A preferred embodiment of the preparation of CLEAs according to the invention is as follows. The enzyme, such as a cofactor dependent enzyme is dissolved in an aqueous medium having a pH of 3 - 10, preferably in the range of 4 - 8, and a temperature of -5°C and 100°C, preferably 0°C and 4°C. The enzyme activity enhancing agent, such as a cofactor, is added wherein the concentration of the enzyme activity enhancing agent is in the range of 1 - 100% to the enzyme (w/w), preferably 1 - 20% (w/w). The precipitant is usually added in steps with stirring and pH control when required. The mixture is allowed to stand for a time, e.g. between 15 min. and 2 hours. Then the crosslinking agent is added to the mixture and again left for some time (e.g. between 30 min. and 12 hours). When the crosslinking reaction is finished the insoluble CLEAs can be filtered or separated by any other method and extensively washed with buffer and/or water before put in a buffered solution for storage.

The invention will further be illustrated by the figure and following examples.

Figure 1 schematically shows parallel enzymatic redox reactions (conversion of the main substrate and cofactor recycling), whereby a second redox reaction is used to regenerate the cofactor using in the reaction cycle of the first enzyme catalyzed process.

### METHODS

Assay of reductive activity of alcohol dehydrogenases.

The reductive activity of horse liver alcohol dehydrogenases was determined by an enzyme-catalysed synthesis of (R)-4-phenyl-2-butanol from benzylacetone. The reductive activity of *Lactobacillus kefir* alcohol dehydrogenases was determined by an enzyme-catalysed synthesis of (R)-1-phenylethanol from acetophenone. The activity of the CLEAs was determined in the presence and/or in the absence of exogenous cofactor in the standard assay. The oxidised cofactor was continuously regenerated by iso-propanol, which was simultaneously present so that the reaction only required catalytic amounts of cofactor. In detail the assay for *Lactobacillus kefir* alcohol dehydrogenase is performed as follows: a certain amount of alcohol dehydrogenase preparation (solid or solution, immobilised or free alcohol dehydrogenase) was added to 1.8 ml phosphate buffer pH 7 containing 1.75 mg NADPH (omitted for the activity test without exogenous cofactor) and 200 µl of 0.1 M acetophenone in iso-propanol. The mixture with a total volume of 2 ml was stirred at 25°C. Samples were taken (100 µl), diluted with 900 µl diisopropyl ether, dried over Na₂SO₄ and analysed by gas chromatography. The assay for horse liver alcohol dehydrogenase is performed as follows: a certain amount of alcohol dehydrogenase preparation (solid or solution, immobilised or free alcohol dehydrogenase) was added to 1.8 ml phosphate buffer pH 7.5 containing 1.7 mg NADH (omitted for the activity test without exogenous cofactor) and 200 µl of 0.1 M benzylacetone in iso-propanol. The mixture with a total volume of 2 ml was stirred at 25°C. Samples were taken (100 µl), diluted with 900 µl diisopropyl ether, dried over Na₂SO₄ and analysed by gas chromatography.

### GC-analysis

The samples were analysed by an Interscience Trace 3000 GC equipped with a CP-chirasil-Dex CB Chrompack Capillary column with a flame ionization detector (250 °C). Nitrogen was used as carrier gas (flow: 14 ml/min). For *Lactobacillus kefir* alcohol dehydrogenase activity assays the column temperature was 105 °C and raised at 2 °C/min to 116 °C. For horse liver alcohol dehydrogenase activity assays the column temperature was 110 °C and raised at 2 °C/min to 125 °C.

### Example 1:

### Influence of NADH on the preparation of horse liver alcohol dehydrogenase CLEAs.

### 1.1 Preparation of enzyme solution

12 mg horse liver alcohol dehydrogenase from Fluka (Switzerland) was dissolved in 1.2 ml 0.1 M potassium phosphate buffer pH 7.5 at 0 °C. A sample was taken for the reference activity test and the solution was divided in 2 portions and NADH was added to the solution as specified in Table 1. The two so prepared solutions are ready for following aggregation.

### 1.2 Preparation of enzyme aggregates

To the two above mentioned clear enzyme solutions were further diluted to 1.6 ml and 1.9 g PEG 6000 was slowly added under stirring at 0°C (in thermostated bath). The mixture was left stirring at 0°C for 2 hours.

### 1.3 Crosslinking enzyme aggregates

Aqueous glutaraldehyde (6 µl, 25% w/w, 2.5 M) was added to the above-prepared solutions to initialise the crosslinking. The mixtures were stirred at 0°C for 2 hours. The reaction mixtures were centrifuged and the activity of the supernatant was measured. The solid CLEAs were dispersed in 1 ml 0.1 M potassium phosphate buffer, pH 7.5 by vortexing. After subsequent centrifugation, the precipitated CLEAs were separated again dispersed in 1 ml 0.1 M potassium phosphate buffer, pH 7.5 by vortexing and the activity of the CLEAs were measured in the presence of exogenous cofactor according to the method section of this patent application.

### 1.4 Results and discussion

It has been found in the experiment that horse liver alcohol dehydrogenase CLEAs were prepared with increased activity when precipitation and crosslinking was performed in the presence of the nicotinamide cofactor NADH. Besides it was found that the aggregate showed increased activity compared to native enzyme when precipitation was performed in the presence of the nicotinamide cofactor NADH (see Table 1). It is apparent that the nicotinamide cofactor stabilises the enzyme and allows access to the enzyme conformation required for the desired reaction.

**Table 1:**

| Influence of NADH on the activity of CLEAs of horse liver alcohol dehydrogenase | | |
|---|---|---|
| Enzyme preparation | NADH added (mg, w/w)) | (% Activity (U/g) |
| Native (reference) | - | 8.8 |
| Aggregate | 1.9 (13) | 18.7 |
| CLEAs | | 13.0 |
| Aggregate | 0 | 1.4 |
| CLEA | | 6.0 |

### Example 2:

### Influence of NADPH on Lactobacillus kefir alcohol dehydrogenase CLEAs

### 2.1 Preparation of the enzyme solution

20 mg *Lactobacillus kefir* alcohol dehydrogenase from Fluka was dissolved in 2 ml 0.1 M potassium phosphate buffer pH 7.0 at 0 °C. The solution was stirred for 15 min. The insoluble components were removed by centrifugation. A sample was taken for the reference activity test. The solution was divided in 2 equal portions and NADPH was added to the clear enzyme solution as specified in Table 2. The so prepared 2 solutions are ready for following aggregation.

### 2.2 Preparation of enzyme aggregates

To the two above mentioned clarified enzyme solutions, 2 ml tert-butyl alcohol was slowly added under stirring at 0 °C (in a thermostated bath). The mixture was left stirring at 0 °C for 2 hours.

### 2.3 Crosslinking enzyme aggregates

Aqueous glutaraldehyde (4.4 µl, 25 w/w %, 2.5M) was added to the above-prepared solutions to initialise the crosslinking. The mixtures were stirred at 0 °C for 2 hours. The reaction mixtures were centrifuged and the activity of the supernatant was measured. The solid CLEAs were dispersed in 1 ml 0.1 M potassium phosphate buffer pH 7.0 by vortexing. After subsequent centrifugation, the precipitated CLEAs were separated again dispersed in 1 ml 0.1 M potassium phosphate pH 7.0 by vortexing and the activity of the CLEAs were measured in the presence of exogenous cofactor according to the method section of this patent application.

### 2.4 Results and discussion

It has been found in the experiment that *Lactobacillus kefir* alcohol dehydrogenase CLEAs were prepared with high activity when precipitation and crosslinking was performed in the presence of nicotinamide cofactor NADPH. When no NADPH was added to the clear enzyme solution, no active aggregates and CLEAs could be prepared (see Table 2).

**Table 2:**

| Influence of NADPH on CLEAs of *Lactobacillus kefir* alcohol dehydrogenase. | | |
|---|---|---|
| Enzyme preparation | NADPH added (mg, %)) | (w/w Activity (U/g) |
| Native (reference) | - | 42.3 |
| Aggregate | 2.6 (15) | 54.7 |
| CLEAs | | 31.5 |
| Aggregate | 0 | 0 |
| CLEAs | | 0 |

### Example 3:

### Effect of crosslinker on NADPH-stabilized Lactobacillus kefir alcohol dehydrogenase CLEAs

### 3.1 Preparation of enzyme solution

70 mg *Lactobacillus kefir* alcohol dehydrogenase in 1.45 ml 0.1 M potassium phosphate buffer pH 7.0 and stirred min at 0 °C for 15. The insoluble components were removed by centrifugation. A sample was taken for the reference activity test. The clear enzyme solution so prepared is ready for following stabilisation and aggregation.

### 3.2 Preparation of enzyme aggregates

From the above-mentioned clarified enzyme solution 135 µl was taken and further diluted with 0.1 M potassium phosphate buffer pH 7.0 to 500 µl. NADPH (1.34 mg, 15 w/w %) was added. Subsequently, tert-butyl alcohol was slowly added under stirring at 0 °C (in a thermostated bath). The mixture was left stirring at 0 °C for 2 hours.

### 3.3 Preparation of enzyme aggregates

Ethylene diamine (0.5 M, pH 6.0, adjusted with 6 M HCl, aqueous solution) and 25 % w/w glutaraldehyde solution (2.5 M) are mixed as specified in Table 4 at 0 °C for 20 min. The so prepared crosslinker was added to the above-prepared enzyme aggregate solution to initialise crosslinking.

### 3.4 Crosslinking enzyme aggregates

After addition of the above-prepared crosslinker, the mixture was stirred at 0 °C (in a thermostated bath) for 2 hours. The solid CLEAs were dispersed in 1 ml 0.1 M potassium phosphate buffer pH 7.0 by vortexing. After centrifugation the precipitated CLEAs were again dispersed in 0.1 ml 0.1 M potassium phosphate buffer, pH 7.0 by vortexing and the activity of the CLEAs were measured without exogenous cofactor, according to the method section of this patent application.

### 3.5 Results and discussion

It has been found in the experiment that the optimal volume ratio of ethylene diamine (0.5 M) /glutaraldehyde (2.5 M) was 4:5 (i.e. a molar ratio of 1.3). The relative activity towards native enzyme is 93% (see Table 3). The activity was determined in the absence exogenous cofactor in the standard activity test. It is believed that the cofactor is bound to the coenzyme-binding domain during aggregation and that after crosslinking its dissociation is prevented. The cofactor is regenerated inside the CLEAs of *Lactobacillus kefir.*

**Table 3:**

| Influence of the ratio of glutaraldehyde/ethylene diamine on the residual activity of CLEAs of *Lactobacillus kefir* alcohol dehydrogenase | | | |
|---|---|---|---|
| Volume (µl) of ethylene diamine to glutaraldehyde for crosslinker preparation | Molar ratio of ethylene diamine to glutaraldehyde | Activity of supernatant after crosslinking (U/g) | Activity of CLEAs (U/g)* |
| Reference (native) # | - | - | 42.3 |
| 0/100 | 0/1 | 0 | 26.5 |
| 50/500 | 1/10 | 0 | 29.5 |
| 100/500 | 1/5 | 0 | 35.4 |
| 100/250 | 1/2.5 | 0 | 35.1 |
| 200/250 | 1/1.3 | 0 | 39.4 |
| 250/250 | 1/1 | 0 | 34.4 |
| 250/200 | 1.3/1 | 0 | 35.2 |
| 200/400 | 2/1 | 0 | 18.9 |

| | | | |
|---|---|---|---|
| * Activity measured in the absence of exogenous cofactor | | | |
| # Activity measured in the presence of exogenous cofactor | | | |

### Example 4:

### Recycle of Lactobacillus kefir alcohol dehydrogenase CLEAs without exogenous cofactor in the activity test

### 4.1 Recycling of Lactobacillus kefir alcohol dehydrogenase CLEAs

The preparations of example 4 were used to test the recyclability of the prepared CLEAs in the absence of exogenous cofactor. The reaction mixture was centrifuged and the supernatant was removed. The remained CLEAs were dispersed in 1 ml 0.1 M potassium phosphate buffer pH 7.0 by vortexing. After centrifugation, the precipitated CLEAs were dispersed in 1 ml 0.1 M potassium phosphate buffer pH 7.0 by vortexing and the activity of the CLEAs was measured without exogenous cofactor according to the method section of this patent application.

### 4.2 Results and discussion

It has been found in the experiment that the CLEAs of lactobacillus kefir alcohol dehydrogenase could be recycled, but loss of activity was observed (Table 4). Therefore the CLEAs are tested in the presence of the exogenous cofactor in the activity test. It is noted that modulation of the composition of the crosslinker systems (ethylene diamine - glutaraldehyde) leads to variation in the performance of the CLEA under recycle conditions, proving the means for preparation of CLEA system which operated without the need for additional co-factor in subsequent processing.

**Table 4:**

| Recycling of *Lactobacillus kefir* alcohol dehydrogenase CLEAs in absence of exogenous cofactor in the activity test | | |
|---|---|---|
| Molar ratio ethylene diamine to glutaraldehyde | Activity of CLEAs (U/g)* | |
| | Run 1 | Run 2 |
| 0/1 | 26.5 | 5.7 |
| 1/10 | 29.5 | 11.5 |
| 1/5 | 35.4 | 11.5 |
| 1/2.5 | 35.1 | 4.2 |
| 1/1.3 | 39.4 | 21.1 |
| 1/1 | 34.4 | 12.3 |
| 1.3/1 | 35.2 | 14.2 |
| 2/1 | 18.9 | 8.9 |

| | | |
|---|---|---|
| * Activity measured in absence of exogenous cofactor | | |

### Example 5:

### Effect of crosslinker on NADPH-stabilized Lactobacillus kefir alcohol dehydrogenase CLEAs with exogenous cofactor in the activity test

### 5.1 Recycling of NADPH-stabilized Lactobacillus kefir alcohol dehydrogenase CLEAs

The preparations of example 4 were used to test the activity of the prepared CLEAs in the presence of the exogenous cofactor in the activity test according to the method section of this patent application.

### 5.2 Results and discussion

It has been found in the experiment that the optimal volume ratio of ethylene diamine (0.5 M) /glutaraldehyde (2.5 M) was 2:5 (i.e. a molar ratio of 1.2.5). The relative activity towards native enzyme is 107 % (Table 5).

**Table 5:**

| Influence of the ratio of glutaraldehyde/ethylene diamine on the residual activity of CLEAs of *Lactobacillus kefir* alcohol dehydrogenase | | | |
|---|---|---|---|
| Volume (µl) of ethylene diamine to glutaraldehyde for crosslinker preparation | Molar ratio of ethylene diamine to glutaraldehyde | Activity of supernatant after crosslinking (U/g) | Activity of CLEAs (U/g)* |
| Reference (native) | - | - | 42.3 |
| 0/100 | 0/1 | 0 | 31.5 |
| 50/500 | 1/10 | 0 | 36.6 |
| 100/500 | 1/5 | 0 | 43.3 |
| 100/250 | 1/2.5 | 0 | 45.0 |
| 200/250 | 1/1.3 | 0 | 44.3 |
| 250/250 | 1/1 | 0 | 40.5 |
| 250/200 | 1.3/1 | 0 | 41.2 |
| 200/400 | 2/1 | 0 | 22.4 |

| | | | |
|---|---|---|---|
| * Activity measured in the presence of exogenous cofactor | | | |

### Example 6:

### Recycle of Lactobacillus kefir alcohol dehydrogenase CLEAs in the presence of exogenous cofactor in the activity test

### 6.1 Recycling of NADPH-stabilized Lactobacillus kefir alcohol dehydrogenase CLEAs

The preparations of example 6 were used to test the recyclability of the prepared CLEAs. The reaction mixture was centrifuged and the supernatant was removed. The precipitated CLEAs were dispersed in 1 ml 0.1 M potassium phosphate buffer pH 7.0 by vortexing. After centrifugation, the remained CLEAs were dispersed in 1 ml 0.1 M potassium phosphate buffer pH 7.0 by vortexing and the activity of the CLEAs was measured in the presence of exogenous cofactor according to the method section of this patent application.

### 6.2 Results and discussion

It has been found in the experiment that the CLEAs of *Lactobacillus kefir* alcohol dehydrogenase could be recycled without a decrease in activity (Table 6).

**Table 6:**

| Recycling of *Lactobacillus kefir* alcohol dehydrogenase CLEAs in the presence of exogenous cofactor in the activity test | | |
|---|---|---|
| Molar ratio ethylene diamine to glutaraldehyde | Activity of CLEAs (U/g)* | |
| | Run 1 | Run 2 |
| 0/1 | 31.5 | 32.0 |
| 1/10 | 36.6 | 39.8 |
| 1/5 | 43.3 | 40.3 |
| ½.5 | 45.0 | 44.0 |
| 1/1.3 | 44.3 | 45.1 |
| 1/1 | 40.5 | 41.9 |
| 1.3/1 | 41.2 | 39.6 |
| 2/1 | 22.4 | 24.8 |

| | | |
|---|---|---|
| * Activity measured in the presence of exogenous cofactor | | |

## Claims

1. Method for the preparation of crosslinked enzyme aggregates, comprising the steps of:
A - providing a plurality of enzyme molecules,
B - aggregating the enzymes molecules in a liquid medium, by means of addition of a precipitating agent,
C - crosslinking the aggregated enzymes to one another by providing a crosslinking agent in the liquid medium, the method comprising, during or between steps A, B or C, the step of adding an enzyme activity enhancing agent, interacting with the said enzyme molecules.

2. Method according to claim 1, wherein the enzyme molecules comprise cofactor dependent enzyme molecules.

3. Method according to claim 1 or 2, wherein the enzyme activity enhancing agent comprises a cofactor.

4. Method according to claim 3, wherein the cofactor is chosen from the group of nicotinamide cofactors, comprising NADH, NAD⁺, NADPH, NADP⁺.

5. Method according to claim 3, wherein the cofactor is an analogue of the nicotinamide cofactor.

6. Method according to any of the preceding claims, wherein the enzyme is an alcohol dehydrogenase.

7. Method according to claim 5, wherein the alcohol dehydrogenase is derived from *Lactobacillus kefir* or horse liver.

8. Method according to any of the preceding claims, wherein the crosslinking agent being prepared by combining a first and a second compound each having at least two reactive groups, the reactive group of the first compound being primary amino groups, the reactive groups of the second compound being aldehyde groups.

9. Method according to any of the preceding claims 2-8, wherein at least two cofactor dependent enzymes are provided in step A) and wherein at least one, preferably more than one, enzyme activity enhancing agents are added during or between steps A), B) or C).

10. Method according to any of the preceding claims, wherein steps B) and C) are performed simultaneously.

11. Method according to any of the preceding claims, further comprising the steps of:
D) performing an enzyme catalysed reaction in a liquid medium in the presence of crosslinked enzyme aggregates, obtained in step C), and
E) recycling the crosslinked enzyme aggregates of step D).

12. Method according to claim 11, wherein steps D) and E) are combined and wherein, in step D), at least two enzyme catalysed reactions are performed and wherein the crosslinked enzyme aggregates comprises at least two cofactor dependent enzymes, the first enzyme being active in catalysing the first reaction when associated with the cofactor in oxidised state, the second enzyme being active in catalysing the second reaction when associated with the said cofactor in reduced state.

13. Method according to any of the preceding claims, wherein enhanced stabilisation of the enzyme system allow for recycle of the CLEA.

14. Crosslinked enzyme aggregates of enzymes, obtainable according to the method according to any of the preceding claims.

15. Crosslinked enzyme aggregates of enzymes comprising an enzyme activity enhancing agent associated therewith.
